# EUROPÄISCHE PATENTSCHRIFT

(11) **EP 3 057 667 B1**
(45) Veröffentlichungstag und Bekanntmachung des Hinweises auf die Patenterteilung: **04.03.2020**
(21) Anmeldenummer: 14796679.0
(22) Anmeldetag: 15.10.2014
(51) Int. Cl.: A61K 47/18, A61K 9/06, A61K 31/566, A61Q 19/00, A61Q 19/06, A61K 8/63, A61K 31/575, A61K 9/00, A61P 17/00

(54) **STEROID-CARBONSÄUREESTER, ZUSAMMENSETZUNGEN, ENTHALTEND STEROID-CARBONSÄUREESTER UND VERWENDUNG DIESER BEI LOKAL TOPISCHER APPLIKATION FÜR KOSMETISCHE ODER DERMATOLOGISCHE ZWECKE**
STEROID CARBOXYLIC ACID ESTERS, COMPOSITIONS CONTAINING STEROID CARBOXYLIC ACID ESTERS, AND USE OF SAID COMPOSITIONS IN LOCAL TOPICAL APPLICATIONS FOR COSMETIC OR DERMATOLOGICAL PURPOSES
ESTERS D'ACIDE STÉROÏDE-CARBOXYLIQUE, COMPOSITIONS CONTENANT DES ESTERS D'ACIDE STÉROÏDE-CARBOXYLIQUE ET LEUR UTILISATION DANS UNE APPLICATION LOCALEMENT TOPIQUE À DES FINS COSMÉTIQUES OU DERMATOLOGIQUES

(30) Priorität: 15.10.2013 DE 102013111391
(43) Veröffentlichungstag der Anmeldung: 24.08.2016
(73) Patentinhaber: CHelac Holding GmbH, 79098 Freiburg (DE)
(72) Erfinder: WIELAND, Heinrich, 79271 St. Peter (DE); KESSEMEIER, Marc, A., 79312 Emmendingen (DE); ZUHSE, Ralf, 12279 Berlin (DE)
(74) Vertreter: Geitz Truckenmüller Lucht Christ
(86) Internationale Anmeldenummer: PCT/DE2014/100366
(87) Internationale Veröffentlichungsnummer: WO 2015/055179

(56) Entgegenhaltungen:
- US-A- 4 177 267
- US-A- 5 807 849
- US-A1- 2003 199 487
- US-A1- 2006 178 352
- US-B1- 6 465 446

## Beschreibung

### Bereich der Erfindung

Die vorliegende Erfindung betrifft die Verwendung eines Steroid-Carbonsäureesters oder einer diesen Carbonsäureester enthaltenden Zusammensetzung bei lokal topischer Applikation für kosmetische oder dermatologische Zwecke. Die Erfindung betrifft auch die Verwendung eines Steroid-Carbonsäureesters oder einer diesen Carbonsäureester enthaltenden Zusammensetzung bei lokal topischer Applikation für kosmetische oder dermatologische Zwecke zur Reduktion von Cellulite, allgemeiner Hautstraffung, Abmilderung von Dehnungsstreifen, Reduktion der Größe von Narben sowohl vor und nach deren Entstehung, Abmilderung von Hautirritationen auf allergischer Basis oder aufgrund von Insektenstichen oder UV- oder Röntgeneinstrahlung und allgemeinem Juckreiz der Haut. Darüber hinaus betrifft die vorliegende Erfindung auch Steroid-Carbonsäureester und Zusammensetzungen, enthaltend diese, zur lokal topischen Applikation.

### Stand der Technik

Es ist bekannt, dass bestimmte Steroidhormone und ihre Metaboliten aufgrund ihrer positiven Einflüsse auf den Körper für kosmetische Zwecke eingesetzt werden können. Beispielsweise steigern Östrogene, sowohl systemisch als auch topisch gegeben, bei postmenopausalen Frauen Dicke und Elastizität der Haut. Die Zunahme der Dicke beruht auf besserer Hydratisierung und auf einer Zunahme der Kollagenkonzentration in der Haut. Der wesentliche Östrogenrezeptor der Haut ist ERbeta. Die topische Applikation östrogenhaltiger Formulierungen zur positiven Beeinflussung der Erscheinungsbildes der Haut und ihrer Eigenschaften führt wegen der hohen Empfindlichkeit des Rezeptors gegenüber Östradiol jedoch zu systemischen und unerwünschten Wirkungen.

WO 97/10255 A1 betrifft bestimmte Steroid-Carbonsäureester und deren Verwendung zur Regulierung von Hautatrophie und anderen Hautzuständen sowie entsprechende kosmetische und pharmazeutische Zubereitungen.

US 4,235,893 A offenbart ein Verfahren zur Inhibierung der Biosynthese des Östrogens und Verwendung bestimmter 4-O-n-Alkanoyl-Androsten-3,17-dione, wobei unter anderem das entsprechende Acetat, das n-Heptanoat und das n-Dodecanoat genannt sind.

US 6,586,417 B1 beschreibt die Verwendung bestimmter Carbonsäureester des 4-Hydroxy-Androsten-3,17-dions zur Regulierung der athletischen Funktion beim Menschen. US 2003/199487 A1 beschreibt für die Regulierung der athletischen Funktion ein Verfahren zur Verabreichung von Ester-Derivaten des 4-Hydroxytestosteron, wobei die Ester-Derivate Acetat, Heptanoat, Decanoat, Hemisuccinat, Benzoat und Propionat einschließen.

US 4 177 267 A beschreibt ein Verfahren zur Verbesserung der Gewebepenetration physiologisch aktiver steroidaler Mittel. Die verbesserte Penetration wird erzielt, indem die Mittel zusammen mit Dimethylsulfoxid verabreicht werden. Zum Auftrag auf das Gewebe werden Lotionen, Salben und Cremes offenbart, die Testosteron-propionat-Derivate enthalten.

US 5 807 849 A betrifft eine therapeutische Methode zur Behandlung von Patienten mit verminderter Produktion an Sexualsteroid-Vorstufen unter Einsatz von Dehydroepiandrosteron und Dehydroepiandrosteronsulfat und deren Derivate. Zur Verabreichung werden Lotionen-, Gel-, Salben- oder Cremeformulierungen genannt. Es wird eine Reihe 3β-Carbonsäureester aufgelistet, von denen erwartet wird, dass sie in vivo in Dehydroepiandrosteron umgesetzt werden. Zudem wird die Herstellung von Dehydroepiandrosteron-3β-undecanoat beschrieben.

US 6 465 446 B1 betrifft die Behandlung von Dermatitis durch die topische Applikation von Δ5-Androsten-3β-ol-7,17 dionen und metabolisierbaren Vorstufen hiervon. Δ5-Androsten-3β-acetyl-7,17 dion wird als solche Vorstufe genannt.

US 2006/178352 A1 befasst sich mit der Verwendung von Androgenen bei der Behandlung von Cellulite. Die Verabreichung der Androgene kann gleichermaßen durch orale Verabreichung wie durch topische Applikation auf die Haut erfolgen. Ein bevorzugtes Steroidderivat ist Testosteron-undecanoat.

### Aufgabe der Erfindung

Es ist daher Aufgabe der Erfindung unerwünschte Steroidwirkungen bei lokal topischer Applikation für kosmetische oder dermatologischer Zwecke zu reduzieren und dabei die Wirkungsspezifität auf bestimmte Zellen der Haut zu erhöhen. Zudem sollen sowohl Cellulite als auch Hautirritationen auf allergischer Basis oder aufgrund von Insektenstichen oder UV- oder Röntgenstrahlung und allgemeinem Juckreiz der Haut positiv beeinflusst werden können.

### Offenbarung der Erfindung

Die Aufgabe wird gelöst durch die Verwendung eines Steroid-4-Carbonsäureesters der Androst-4-en-dione,
mit einer Acylgruppe des Carbonsäureesters 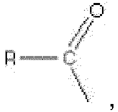
wobei R ausgewählt ist aus Alkyl mit mindestens zwei Kohlenstoffatomen oder Cycloalkyl,
oder Verwendung einer diese Steroid-Carbonsäureester enthaltenden Zusammensetzung bei lokal topischer Applikation für kosmetische oder dermatologische Zwecke.

Die vorliegende Erfindung beruht auf der Beobachtung, dass bei der Verwendung bestimmter Steroid-Carbonsäureester oder einer diese enthaltende Zusammensetzung bei lokal topischer Applikation auf die Haut die Steroid-Carbonsäureester im Gegensatz zu den Steroiden mit freien Hydroxylgruppen lediglich in bestimmten Hautzellen ihre Wirkung entfalten, in anderen Zellen der Haut jedoch nicht.

Bei topischer Applikation dringen die Steroid-Carbonsäureester zunächst schnell in das Stratum Corneum (Hornhautschicht) der Epidermis ein. Von dieser Schicht gelangen die Steroid-Carbonsäureester in die tieferliegenden Keratinozytenschichten der Epidermis und in die Fibroblasten der Dermis. Es wurde gefunden, dass Steroid-4-Carbonsäureester der Androst-4-en-dione, mit einer Acylgruppe des Carbonsäureesters 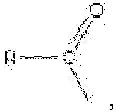 wobei R ausgewählt ist aus Alkyl mit mindestens zwei Kohlenstoffatomen oder Cycloalkyl in der Haut des Menschen keine biologische Wirkung aufweisen. Eine biologische Wirkung entfaltet sich erst nach der Hydrolyse des Steroid-Carbonsäureesters in das freie, hydroxylierte Steroid und die Carbonsäure.

Die Hydrolyse des Steroid-Carbonsäureesters findet in den Keratinozyten statt. Das Enzym, welches die Hydrolyse des Steroid-Carbonsäureesters katalysiert, ist die humane Carboxylesterase 2 (hCES2). Der Anteil an Steroid-Carbonsäureester, der nicht in der Keratinozytenschicht hydrolysiert wird und in die Dermis gelangt, bleibt unwirksam, da die Esterase nahezu ausschließlich in der Epidermis angesiedelt ist. Der Anteil an Steroid-Carbonsäureester, die nicht in der Keratinozytenschicht hydrolysiert wird und in die Dermis und damit in die Fibroblasten gelangt, bleibt unwirksam. Hierdurch kann eine sehr spezifische Wirkung von Steroiden gezielt in den Keratinozyten freigesetzt werden, während andererseits unerwünschte Wirkungen in anderen Zellen, wie beispielsweise den Fibroblasten vermieden werden.

Ein weiterer Vorteil der Steroid-Carbonsäureester liegt darin, dass diese schneller in das Stratum Corneum und das darunter liegende Stratum Lucidum eindringen als die entsprechend unveresterten Steroide. Die Veresterung der Hydroxylgruppen von Steroiden mit Carbonsäuren verringert deren Wasserlöslichkeit. Werden die Steroid-Carbonsäureester auf die Haut aufgetragen, lösen sie sich besser in den Lipiden des Stratum Corneum und Stratum Lucidum als das unveresterte Steroid. Stratum Corneum und Stratum Lucidum grenzen an das Stratum Granulosum, das ebenfalls wie die darunter liegenden Schichten, Stratum spinosum und Stratum Basale der Epidermis, überwiegend aus Keratinozyten besteht. Diese Zellen werden von der Interzellulärflüssigkeit umspült. Da die Steroid-Carbonsäureester hydrophober sind, dringen sie anschließend langsamer in die Keratinozytenschicht, die als wässriges Milieu angesehen werden kann, ein. Die weitgehend wasserunlöslichen Steroid-Carbonsäureester treten nur sehr verzögert aus dem Stratum Corneum in das Stratum Basale über, so daß die Kombination von relativ leichtem Eindringen in die sich zwischen den Hornzellen befindlichen Lipide und dem verlangsamten Austritt in die Interzellulärflüssigkeit zu einer Anreicherung der Steroid-Carbonsäureester im Stratum Corneum führt (Stratum-Corneum-Reservoir). Dieses Reservoir gibt die gespeicherten Steroid-Carbonsäureester stetig aber langsam ab. Es kommt also zu einem weniger raschen Anfluten von Steroiden zu den Keratinozyten nach topischer Applikation auf die Haut, wenn es sich dabei um Steroid-Carbonsäureester statt um Steroide mit freien Hydroxylgruppen wie Östradiol oder Testosteron handelt. Diese haben eine längere Verweildauer in der Haut als die freien Hydroxyl-Steroide. Damit entsteht ein Reservoireffekt, der beinhaltet, daß die Steroid-Carbonsäureester kontinuierlich an die Epidermis abgegeben werden. Damit ist auch der Konzentrationsgipfel der Substanzen in der Keratinozytenschicht geringer und zeitlich später als bei den hydroxylierten, unveresterten Steroiden.

Ein weiterer Gegenstand der Erfindung ist die Verwendung eines Steroid-4-Carbonsäureesters der Androst-4-en-dione,
mit einer Acylgruppe des Carbonsäureesters 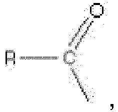
wobei R ausgewählt ist aus Alkyl mit mindestens zwei Kohlenstoffatomen oder Cycloalkyl,
oder Verwendung einer diese Steroid-Carbonsäureester enthaltenden Zusammensetzung bei lokal topischer Applikation für kosmetische und dermatologische Zwecke zur Reduktion von Cellulite, zur allgemeinen Hautstraffung, Abmilderung von Dehnungsstreifen, Reduktion der Größe von Narben sowohl vor und nach deren Entstehung, Abmilderung von Hautirritationen auf allergischer Basis oder aufgrund von Insektenstichen oder UV- oder Roentgeneinstrahlung und allgemeinem Juckreiz der Haut.

Bei der Verwendung eines Steroid-Carbonsäureesters für die topische Applikation auf die Haut wurde beobachtet, dass eine Verbesserung der Reduktion von Cellulite, eine verbesserte allgemeine Hautstraffung, eine verbesserte Abmilderung von Dehnungsstreifen, eine Verbesserung der Reduktion der Größe von Narben sowohl vor und nach deren Entstehung, eine verbesserte Abmilderung von Hautirritationen auf allergischer Basis oder aufgrund von Insektenstichen oder UV- oder Roentgeneinstrahlung und allgemeinem Juckreiz der Haut im Vergleich mit den unveresterten Steroiden auftrat.

In den Fibroblasten findet die Kollagensynthese der Dermis statt. In diesen Zellen befindet sich das Enzym Aromatase. Dieses Enzym ist maßgeblich an der Östrogensynthese der Haut beteiligt. Die Östrogene binden an den Östrogenrezeptor-beta (ERbeta). Dies führt zu einer vermehrten Kollagensynthese in den Fibroblasten. Es ist bekannt, dass zwischen der Aromataseaktivität in den Fibroblasten und der Reduktion von Cellulite, einer allgemeinen Hautstraffung, einer Abmilderung von Dehnungsstreifen und einer Reduktion der Größe von Narben sowohl vor und nach deren Entstehung, eine positive Korrelation besteht. Es hat sich überraschend gezeigt, dass Hautirritationen auf allergischer Basis oder aufgrund von Insektenstichen oder UV- oder Roentgeneinstrahlung und allgemeinem Juckreiz der Haut durch die topische Applikation sehr günstig beeinflusst werden können.

Bei vielen Steroiden aus der Gruppe der Androstanone, der Androst-4-en-dione, der Androst-5-en-dione, der Dehydroepiandrosterone, der Androstentrione oder der Testosterone handelt es sich um Aromatase-Inhibitoren. Bei Verwendung dieser zur topischen Applikation auf der Haut wirken sich diese Steroide nachteilig auf die Östrogensynthese aus.

In den Kerationozyten werden jedoch aus den Aromatase-Inhibitoren in hohem Maß Stoffwechselprodukte erzeugt, die die Aromatase im Gegensatz zu ihren Vorstufen nicht mehr hemmen und die darüber hinaus die Kollagensynthese in den Fibroblasten fördern, da sie wie die Östrogene auch an den Östrogenrezeptor-beta (ERbeta) der Fibroblasten binden. In den Kerationozyten entstehen demnach aus den Aromatase-Inhibitoren in hohen Kozentrationen Liganden für den Östrogenrezeptor-beta. Diese Stoffwechelprodukte der Keratinozyten wirken parakrin auf die Kollagensynthese der Fibroblasten. Die unveresterten Steroide aus der Gruppe der Androstanone, der Androst-4-en-dione, der Androst-5-en-dione, der Dehydroepiandrosterone, der Androstentrione oder der Testosterone besitzen daher sowohl positive als auch negative Auswirkungen.

Durch die Verwendung von Steroid-Carbonsäureester ist es im Gegensatz dazu möglich, die unerwünschte, den positiven Effekt der Steroide verringernde Wirkung, zu vermeiden. Hierdurch wird der positive Effekt der Steroide auf den Östrogenrezeptor-beta und damit die mit der Aktivierung dieses Rezeptors verbundenen Wirkungen hinsichtlich der Reduktion von Cellulite, einer allgemeinen Hautstraffung, einer Abmilderung von Dehnungsstreifen, einer Reduktion der Größe von Narben sowohl vor und nach deren Entstehung, einer Abmilderung von Hautirritationen auf allergischer Basis oder aufgrund von Insektenstichen oder UV- oder Roentgeneinstrahlung und allgemeinem Juckreiz der Haut verbessert.

Der Steroid-Carbonsäureester ist ein Derivat des 4-Hydroxyandrostendions. Der Steroid-4-Carbonsäureester des Androstendions wird in den Keratinozyten gespalten. In den Keratinozyten entsteht im Weiteren aus 4-Hydroxyandrostendion als wesentlicher Metabolit eine Verbindung, die an C3 nicht mehr die Keto-gruppe des Androstendions, sondern eine Hydroxylgruppe aufweist. Diese Verbindung besitzt darüber hinaus keine C4=C5 Doppelbindung mehr. 70 % der Hydroxylgruppen an C3 befinden sich in beta-Position. Das Molekül ist also 3beta,4beta-Dihydroxy-5alpha-Androstan-17-on. In den Keratinozyten wird zudem noch die Keto-Gruppe an C-17 reduziert, und es entsteht ein Triol. Dieses bindet sich sehr gut an den Östrogenrezeptor-beta (ERbeta). Steroid-Carbonsäureester des 4-Hydroxyandrostendions bewirken eine sehr effiziente Reduktion von Cellulite.

Nach einer vorteilhaften Ausgestaltung der Erfindung ist die Acylgruppe des Carbonsäureesters frei von polaren Substituenten. Der Rest R, der ausgewählt ist aus Alkyl mit mindestens zwei Kohlenstoffatomen oder Cycloalkyl, weist keine polaren Substituenten auf. Für die Ausbildung eines Stratum-Corneum-Reservoirs ist die geringere Wasserlöslichkeit des Steroid-Carbonsäureesters im Vergleich zu den unveresterten Steroiden bedeutend. Hydroxylgruppen oder andere polare Substituenten an der Acylgruppe wirken sich negativ auf die Bildung des Reservoir-Effekts aus, da der Rest R durch die polaren Substituenten einen hydrophileren Charakter erhalten würde. Die Einflüsse eines polaren und damit hydrophilen Substituenten müssten gegebenenfalls wieder durch weitere hydrophobe Einheiten ausgeglichen werden. Hydroxylgruppen und andere polare Substituenten der Acylgruppe des Carbonsäureesters wie beispielsweise Aminogruppen oder Carboxylgruppen, sind daher nicht erwünscht.

Nach einer weiteren vorteilhaften Ausgestaltung der Erfindung ist der Alkylrest der Acylgruppe unverzweigt oder verzweigt. Wird der Alkylrest hierbei aus Ethyl, Propyl, Isopropyl, Butyl, sec-Butyl (1-Methylpropyl), Isobutyl (2-Methylpropyl), tert-Butyl (1,1-Dimethylethyl), Pentyl, Hexyl sowie Strukturisomeren des Pentyl oder Hexyl ausgewählt, ist eine sehr gute Hydrolyserate der Steroid-Carbonsäureester durch die hCES2 der Kerationozyten zu beobachten.

Nach einer weiteren vorteilhaften Ausgestaltung der Erfindung ist der Cycloalkylrest ausgewählt aus Cyclopropan, Cyclopentan oder Cyclohexan. Diese Cycloalkyle werden durch die hCES2 der Kerationozyten mit einer sehr guten Hydrolyserate von den entsprechenden Steroid-Carbonsäureestern abgespalten.

Nach einer weiteren vorteilhaften Ausgestaltung der Erfindung ist der Steroid-Carbonsäureester 4-O-Propionyl-Androsten-3,17-Dion, 4-O-Butyryl-Androsten-3,17-Dion, 4-O-Valeryl-Androsten-3,17-Dion, 4-O-Caproyl-Androsten-3,17-Dion oder 4-O-Enantyl-Androsten-3,17-Dion. Die Steroid-Carbonsäureester weisen sowohl eine ausgezeichnete Hydrolyserate durch die hCES2 der Kerationozyten auf als auch eine ausgeprägte Aktivierung des Östrogenrezeptors-beta (ERbeta) durch ihre in den Kerationozyten gebildeten Metabolite. Insbesondere bei einer Verwendung von 4-O-Propionyl-Androst-4-en-3,17-Dion wurden besonders gute Ergebnisse hinsichtlich der Reduktion von Cellulite, einer allgemeinen Hautstraffung, einer Abmilderung von Dehnungsstreifen, einer Reduktion der Größe von Narben sowohl vor und nach deren Entstehung, einer Abmilderung von Hautirritationen auf allergischer Basis oder aufgrund von Insektenstichen oder UV- oder Roentgeneinstrahlung und allgemeinem Juckreiz der Haut erzielt.

Nach einer weiteren vorteilhaften Ausgestaltung der Erfindung ist der Steroid-Carbonsäureester in einer Zusammensetzung in einer Menge von 0,001 bis 10 Gew.% bezogen auf die Gesamtmenge der Zusammensetzung enthalten. Der Gehalt der Wirksubstanz wird hierbei auf den jeweiligen Anwendungsfall angepasst. Geeignet sind vorzugsweise Wirkstoffgehalte der Steroid-Carbonsäureester in der gesamten Zusammensetzung von 0,001 bis 5 Gew.% und insbesondere 0,3 bis 2 Gew.%
Nach einer weiteren vorteilhaften Ausgestaltung der Erfindung ist der Steroid-Carbonsäureester in einer Zusammensetzung enthalten, die als Salbe, Creme, Gel, Öl oder Emulsion bzw. Lotion formuliert ist. Hierdurch wird die topische Anwendung erleichtert. Zu diesem Zweck erfasst die Zusammensetzung Zusatzstoffe, die für die entsprechende Formulierung als Salbe, Creme, Gel, Öl oder Emulsion bzw. Lotion üblich sind. Die weiteren, gegebenenfalls vorhandenen Zusatzstoffe können in den für die jeweilige Formulierungen üblichen Mengen eingesetzt werden.

Weiterhin beschrieben ist ist 4-O-Propionyl-Androst-4-en-3,17-Dion mit der Formel 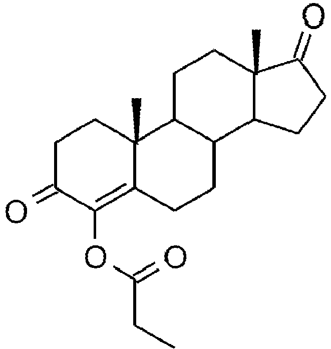 zur Verwendung bei lokal topischer Applikation für kosmetische oder dermatologische Zwecke.

Es hat sich gezeigt, dass bei topischer Applikation des 4-O-Propionyl-Androst-4-en-3,17-Dion auf die Haut eine Verbesserung der Reduktion von Cellulite, eine verbesserte allgemeine Hautstraffung, eine verbesserte Abmilderung von Dehnungsstreifen, eine Verbesserung der Reduktion der Größe von Narben sowohl vor und nach deren Entstehung, eine verbesserte Abmilderung von Hautirritationen auf allergischer Basis oder aufgrund von Insektenstichen oder UV- oder Roentgeneinstrahlung und allgemeinem Juckreiz der Haut auftrat.

Ein weiterer Erfindungsgegenstand ist eine Zusammensetzung zur Verwendung bei lokal topischer Applikation für kosmetische oder dermatologische Zwecke, die mindestens einen Steroid-4-Carbonsäureester der Androst-4-en-dione, mit einer Acylgruppe des Carbonsäureesters 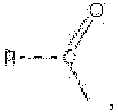 wobei R ausgewählt ist aus Alkyl mit mindestens zwei Kohlenstoffatomen oder Cycloalkyl, in einer Formulierung als Salbe, Creme, Gel, Öl, Emulsion oder Lotion enthält.

Die Zusammensetzung ist besonders geeignet bei lokal topischer Applikation für kosmetische und dermatologische Zwecke zur Reduktion von Cellulite, zur allgemeinen Hautstraffung, Abmilderung von Dehnungsstreifen, Reduktion der Größe von Narben sowohl vor und nach deren Entstehung, Abmilderung von Hautirritationen auf allergischer Basis oder aufgrund von Insektenstichen oder UV- oder Roentgeneinstrahlung und allgemeinem Juckreiz der Haut. Es hat sich gezeigt, dass bei topischer Applikation der Zusammensetzung auf die Haut eine Verbesserung der Reduktion von Cellulite, eine verbesserte allgemeine Hautstraffung, eine verbesserte Abmilderung von Dehnungsstreifen, eine Verbesserung der Reduktion der Größe von Narben sowohl vor und nach deren Entstehung, eine verbesserte Abmilderung von Hautirritationen auf allergischer Basis oder aufgrund von Insektenstichen oder UV- oder Roentgeneinstrahlung und allgemeinem Juckreiz der Haut auftrat.

Der Steroid-Carbonsäureester ist ein Derivat des 4-Hydroxyandrostendions. Bevorzugt ist der Rest R der Acylgruppe des Carbonsäureesters ein Alkylrest mit mindestens zwei Kohlenstoffatomen und unverzweigt oder verzweigt. Vorteilhafterweise ist der Alkylrest aus Ethyl, Propyl, Isopropyl, Butyl, sec-Butyl (1-Methylpropyl), Isobutyl (2-Methylpropyl), tert-Butyl (1,1-Dimethylethyl), Pentyl, Hexyl sowie Strukturisomeren des Pentyl oder Hexyl ausgewählt. Eine andere bevorzugte Ausführungsform sieht vor, dass der Rest R der Acylgruppe ein Cycloalkylrest ist und der Cycloalkylrest ausgewählt ist aus Cyclopropan, Cyclopentan oder Cyclohexan.

Nach einer vorteilhaften Ausgestaltung der Zusammensetzung ist die Acylgruppe des Carbonsäureesters frei von polaren Substituenten.

Nach einer weiteren vorteilhaften Ausgestaltung der Zusammensetzung ist der Steroid-Carbonsäureester 4-O-Propionyl-Androsten-3,17-Dion, 4-O-Butyryl-Androsten-3,17-Dion, 4-O-Valeryl-Androsten-3,17-Dion, 4-O-Caproyl-Androsten-3,17-Dion oder 4-O-Enantyl-Androsten-3,17-Dion. Die Steroid-Carbonsäureester weisen sowohl eine ausgezeichnete Hydrolyserate durch die hCES2 der Kerationozyten auf als auch eine ausgeprägte Aktivierung des Östrogenrezeptors-beta (ERbeta) durch ihre in den Kerationozyten gebildeten Metabolite.

Nach einer weiteren vorteilhaften Ausgestaltung der Zusammensetzung ist der Steroid-Carbonsäureester in der Zusammensetzung in einer Menge von 0,001 bis 10 Gew.% bezogen auf die Gesamtmenge der Zusammensetzung enthalten.

Nach einer weiteren vorteilhaften Ausgestaltung der Zusammensetzung ist der Steroid-Carbonsäureester in der Zusammensetzung in einer Menge von 0,001 bis 5 Gew.% bezogen auf die Gesamtmenge der Zusammensetzung enthalten.

Die erfindungsgemäße Zusammensetzung kann kosmetische Zusatzstoffe, die für die entsprechende Formulierung als Salbe, Creme, Gel, Öl oder Emulsion bzw. Lotion üblich sind, wie beispielsweise Fette, Öle, Wachse, Alkohole, Polyole, Polymere, Entschäumer, Farbstoffe, Pigmente, Verdickungsmittel, oberflächenaktive Substanzen, Emulgatoren, weichmachende Substanzen, Konservierungsmittel, Antioxidantien, Puffersubstanzen, Duftstoffe und/oder Parfüm in den für die jeweilige Formulierungen üblichen Mengen enthalten.

Die folgenden Beispiele sollen die Erfindung erläutern, aber nicht einschränken. Sofern nichts Anderes angegeben ist, beziehen sich die Zahlenangaben auf Gew.-%.

### Beispiel 1

### Formulierung und Herstellung einer erfindungsgemäßen Emulsion

| **Phase** | **InCI-Bezeichnung** | **Gew.-%** |
|---|---|---|
| A | Aqua/Wasser | 77,9968 |
| | Dinatrium-EDTA | 0,1000 |
| | Panthenol | 0,1000 |
| | Sorbit | 0,4000 |
| | Xanthan | 0,5000 |
| | Glycerin | 5,0000 |
| | Butylenglykol | 1,6000 |
| | Ethylhexylglycerin | 0,4000 |
| B | Cetylalkohol | 2,0000 |
| | Dimethicon | 1,1000 |
| | Kaliumcetylphosphat | 0,7000 |
| | C20-22 Alkylphosphat | 1,8000 |
| | C20-22 Alkylalkohole | 1,5000 |
| | Capryl/Caprintriglycerid | 3,0000 |
| C | Propylenglykol | 1,0000 |
| | Chlorphenesin | 0,2000 |
| | Phenoxyethanol | 0,7000 |
| D | Jojobaöl | 0,5000 |
| | Tocopherylacetat | 0,0500 |
| | Lecithin | 0,0210 |
| | Tocopherol | 0,0045 |
| | Ascorbylpalmitat | 0,0030 |
| | Citronensäure | 0,0015 |
| | Ascorbyltetraisopalmitat | 0,1000 |
| E | Phytinsäure | 0,0800 |
| | Milchsäure | 0,4500 |
| | Natriumhydroxid | 0,2400 |
| F | 4-O-Propionyl-androst-4-en-3,17-dion | 0,2000 |
| G | Duftstoffe/Parfüm | 0,2500 |
| H | Karamell | 0,0032 |

### Ansatz:

Die Inhaltsstoffe der Phase A werden gemischt und unter Rühren auf 70 bis 75°C erhitzt. Die Inhaltsstoffe der Phase B werden gemischt, auf 70 bis 75°C erhitzt und unter Rühren zu der Phase A zugefügt. Die Mischung aus A und B wird unter Rühren auf 50°C abgekühlt. Phase C wird unter weiterem Rühren zugefügt. Die Inhaltsstoffe der Phase D werden gemischt und unter Rühren der Phase aus A+B+C zugefügt. Die Inhaltsstoffe der Phase E werden gemischt und unter Rühren der Phase aus A+B+C+D zugefügt. Phase F wird der Phase aus A+B+C+D+E unter Rühren bei einer maximalen Temperatur von 30 bis 35°C zugemischt. Phase G wird der Phase aus A+B+C+D+E+F unter Rühren bei einer maximalen Temperatur von 25°C zugemischt. Phase H wird der Phase aus A+B+C+D+E+F+G unter Rühren bei einer maximalen Temperatur von 25°C zugemischt. Alle Schritte des Ansatzes können zur Erzielung besonders gleichmäßiger, luftblasenfreier Zusammensetzungen in einem Vakuum-Mischer durchgeführt werden.

### Beispiel 2

### Formulierung und Herstellung einer erfindungsgemäßen Creme

| **Phase** | **InCI-Bezeichnung** | **Gew.-%** |
|---|---|---|
| A | Aqua/Wasser | 78,9830 |
| | Dinatrium-EDTA | 0,1500 |
| | Sorbit | 0,4000 |
| | Glycerin | 2,0000 |
| | Ethylhexylglycerin | 0,4000 |
| | Xanthan | 0,3000 |
| B | Cetearylalkohol | 1,9000 |
| | Capryl/Caprintriglycerid | 1,7000 |
| | Cetearyglukosid | 0,7900 |
| | Glycerylstearat | 0,9000 |
| | Ethylhexylpalmitat | 1,6000 |
| | Cetylpalmitat | 1,6000 |
| | Dicaprylylcarbonat | 2,0000 |
| | Sheabutter | 0,5000 |
| | Bienenwachs weiß - Cera alba | 0,3000 |
| | Natriumpolyacrylat | 1,0000 |
| | PEG-100-Stearat | 0,9000 |
| | hydriertes Polydecen | 0,8000 |
| | Trideceth-6 | 0,1600 |
| C | Chlorphenesin | 0,2000 |
| | Phenoxyethanol | 0,7000 |
| D | Jojobaöl | 0,5000 |
| | Tocopherylacetat | 0,1000 |
| | Lecithin | 0,0350 |
| | Tocopherol | 0,0075 |
| | Ascorbylpalmitat | 0,0050 |
| | Citronensäure | 0,0025 |
| E | Milchsäure | 0,9000 |
| | Natriumhydroxid | 0,6000 |
| F | Phytinsäure | 0,2000 |
| G | Ascorbyltetraisopalmitat | 0,1000 |
| H | 4-O-Propionyl-androst-4-en-3,17-dion | 0,1000 |
| I | Duftstoffe/Parfüm | 0,1670 |

### Ansatz:

Die Inhaltsstoffe der Phase A werden gemischt und unter Rühren auf 70 bis 75°C erhitzt. Die Inhaltsstoffe der Phase B werden gemischt, auf 70 bis 75°C erhitzt und unter Rühren zu der Phase A zugefügt. Die Mischung aus A und B wird unter Rühren auf 50°C abgekühlt. Phase C wird unter weiterem Rühren zugefügt. Die Inhaltsstoffe der Phase D werden gemischt und unter Rühren der Phase aus A+B+C zugefügt. Die Inhaltsstoffe der Phase E werden gemischt und unter Rühren der Phase aus A+B+C+D zugefügt. Phase F wird der Phase aus A+B+C+D+E unter Rühren bei einer maximalen Temperatur von 35 bis 40°C zugemischt. Phase G wird der Phase aus A+B+C+D+E+F unter Rühren bei einer maximalen Temperatur von 35 bis 40°C zugemischt. Phase H wird der Phase aus A+B+C+D+E+F+G unter Rühren bei einer maximalen Temperatur von 30 bis 35°C zugemischt. Phase I wird der Phase aus A+B+C+D+E+F+G+H unter Rühren bei einer maximalen Temperatur von 25°C zugemischt. Alle Schritte des Ansatzes können zur Erzielung besonders gleichmäßiger, luftblasenfreier Zusammensetzungen in einem Vakuum-Mischer durchgeführt werden.

### Beispiel 3

### Formulierung einer weiteren erfindungsgemäßen Creme

| **Bezeichnung** | **Gew.-%** |
|---|---|
| Aqua/Wasser | 56,8 |
| Propylenglykol | 25,0 |
| Isopropylmyristat | 6,0 |
| Cetylstearylalkohol | 6,0 |
| Stearylalkohol | 2,0 |
| Polysorbat 80 (Polyoxyethylen(20)-sorbitan-monooleat) | 2,0 |
| Sorbitanmonostearat | 1,0 |
| Glycerolmonostearat | 1,0 |
| 4-O-Propionyl-androst-4-en-3,17-dion | 0,1 |
| Hyaluronsäure | 0,1 |

### Beispiel 4

### Formulierung einer weiteren erfindungsgemäßen Creme

| **InCI-Bezeichnung** | **Gew.-%** |
|---|---|
| Aqua/Wasser | 62,85 |
| Jojobaöl | 6,05 |
| Cocoglyceride | 5,95 |
| Dimethylisosorbid | 5,00 |
| Glycerin | 4,20 |
| Sorbit | 4,00 |
| Sheabutter | 3,00 |
| Polyglyceryl-3-methylglucosedistearat | 3,00 |
| Cetearylalkohol | 2,20 |
| Bienenwachs gelb - Cera flava | 1,00 |
| Tocopherylacetat | 1,00 |
| Polysol AC: | 0,70 |
| Phenoxyethanol | |
| Dehydroacetsäure | |
| Sorbinsäure | |
| Benzoesäure | |
| Milchsäure | |
| 4-O-Propionyl-androst-4-en-dion | 0,09 |
| Xanthan | 0,25 |
| Duftstoffe/Parfüm | 0,20 |
| Tetranatriumimminodisuccinat | 0,10 |
| Ethylhexylglycerin | 0,10 |
| Milchsäure, 80%ig | 0,10 |
| Ascorbyltetraisopalmitat | 0,10 |
| Phytinsäure | 0,05 |
| Controx VP: | 0,04 |
| Lecithin | |
| Ascorbylpalmitat | |
| hydriertes Palmglyceride Citrat | |
| Tocopherol | |
| Natriumhydroxid | 0,02 |

### Beispiel 5

### Formulierung eines erfindungsgemäßen Olio-Gels

| **Bezeichnung** | **Gew.-%** |
|---|---|
| Oliven-Fruchtöl | 45,37 |
| Capryl/Caprintriglyceride | 31,40 |
| Glycerin | 15,00 |
| Polyglyceryl-5-Oleat | 6,00 |
| Aqua/Wasser | 1,88 |
| 4-O-Propionyl-androst-4-en-3,17-dion | 0,15 |
| Tocopherol, Sonnenblumenöl | 0,20 |

### Ergebnisse einer klinischen Studie mit einer 4-O-Propionyl-androst-4-en-3,17-dion enthaltenden Creme - Wirksamkeit bei Cellulite

In einer von einem unabhängigen Dermatologischen Institut (Dermatest, Münster) durchgeführten Studie wurde eine Creme, die 0,10% 4-O-Propionyl-androst-4-en-3,17-dion enthält bei Probandinnen auf Wirksamkeit und Verträglichkeit getestet.

### Studienprotokoll:

An der Studie nahmen 20 Probandinnen (Alter zwischen 32 und 58 Jahren mittleres Alter 45,1) teil. Eine Probandin beendete die Studie aufgrund einer Schwangerschaft nach 8 Wochen vorzeitig; eine Probandin (21. Probandin) wurde zu Beginn der neunten Woche aufgenommen.

Die Creme wurde über einen Zeitraum von 12 Wochen einmal täglich angewendet. Die Überprüfung der Wirksamkeit und der Verträglichkeit (Hautelastizität, Oberschenkelumfang, Breite und Höhe der protrudierenden Lobuli) erfolgte nach 4, 8 und 12 Wochen.

### Wirksamkeit:

### Hautelastizität

Die Hautelastizität wurde mit einem Cutometer bestimmt. Die Hautelastizität nahm bei 100% der Probandinnen zu. Nach 4 Wochen durchschnittlich um 14%, nach 12 Wochen um durchschnittlich 22,42%. 95% der Probandinnen empfanden subjektiv eine Verbesserung der Hautelastizität.

### Fläche der Fettlobuli

Eine erstaunliche Wirkung wurde hinsichtlich der Größe der protrudierenden Fettlobuli, die für das charakteristische Erscheinungsbild der Cellulite verantwortlich sind, beobachtet. Die der Größe entsprechende Fläche wurde mit Hilfe der Ultraschalluntersuchung vermessen.

Die Fläche der Fettlobuli nahm bei 100% der Probandinnen ab. Nach 4 Wochen nahm die Fläche der Fettlobuli um durchschnittlich 23,24% ab, nach 8 Wochen betrug die Flächenreduktion durchschnittlich 34,51% und nach 12 Wochen betrug die durchschnittliche Abnahme der Fettlobulifläche 44,06%.
Nach Aussagen des unabhängigen Untersuchungsinstitutes wurde bisher eine solche Wirkung noch bei keinem Konkurrenzprodukt beobachtet.

### Oberschenkelumfang

Der Oberschenkelumfang nahm bei 100% der Probandinnen ab. Nach 4 Wochen nahm der Oberschenkelumfang der Probandinnen um durchschnittlich 0,6 cm bzw. 1,04% ab, nach 12 Wochen betrug die durschnittliche Abnahme des Oberschenkelumfanges 1,87 cm bzw. 3,25%.

### Weitere Optionen des Einsatzes in kosmetischen Produkten

a) Dehnungsstreifen/Schwangerschaftsstreifen
b) Falten (Anti-Aging)
c) Hautpflege nach Strahlentherapie (onkologische Pflege)

Die Produktideen Behandlung von Dehnungsstreifen/Schwangerschaftstreifen und Narbenpflege sind jeweils das Resultat von wiederholten positiven Beobachtung bei dem Einsatz der 4-O-Propionyl-Androst-4-en-3,17-Dion enthaltenden Creme; bei allen drei Anwendungen erzielte die 4-O-Propionyl-Androst-4-en-3,17-Dion enthaltende Creme deutlich bessere Ergebnisse, als sie mit auf dem Markt befindlichen Produkten erreicht werden konnten.

Sämtliche Merkmale der Erfindung können sowohl einzeln als auch in beliebiger Kombination miteinander erfindungswesentlich sein. Weitere Vorteile und vorteilhafte Ausgestaltungen der Erfindung sind den Ansprüchen zu entnehmen.

## Patentansprüche

1. Verwendung eines Steroid-4-Carbonsäureesters der Androst-4-en-dione, mit einer Acylgruppe des Carbonsäureesters 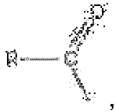
wobei R ausgewählt ist aus Alkyl mit mindestens zwei Kohlenstoffatomen oder Cycloalkyl,
oder Verwendung einer diese Steroid-Carbonsäureester enthaltenden Zusammensetzung bei lokal topischer Applikation für kosmetische Zwecke, **dadurch gekennzeichnet, dass** der Steroid-Carbonsäureester ein Derivat des 4-Hydroxyandrostendions ist.

2. Verwendung nach Anspruch 1, **dadurch gekennzeichnet, dass** die Acylgruppe des Carbonsäureesters frei von polaren Substituenten ist.

3. Verwendung nach einem der vorhergehenden Ansprüche, **dadurch gekennzeichnet, dass** der Rest R der Acylgruppe ein Alkylrest mit mindestens zwei Kohlenstoffatomen ist und der Alkylrest unverzweigt oder verzweigt ist.

4. Verwendung nach einem der vorhergehenden Ansprüche, **dadurch gekennzeichnet, dass** der Rest R der Acylgruppe ein Alkylrest mit mindestens zwei Kohlenstoffatomen ist und der Alkylrest ausgewählt ist aus Ethyl, Propyl, Isopropyl, Butyl, *sec*-Butyl (1-Methylpropyl), Isobutyl (2-Methylpropyl), tert-Butyl (1,1-Dimethylethyl), Pentyl, Hexyl sowie Strukturisomeren des Pentyl oder Hexyl.

5. Verwendung nach einem der vorhergehenden Ansprüche, **dadurch gekennzeichnet, dass** der Rest R der Acylgruppe ein Cycloalkylrest ist und der Cycloalkylrest ausgewählt ist aus Cyclopropan, Cyclopentan oder Cyclohexan.

6. Verwendung nach einem der vorhergehenden Ansprüche, **dadurch gekennzeichnet, dass** der Steroid-Carbonsäureester 4-O-Propionyl-Androsten-3,17-Dion, 4-O-Butyryl-Androsten-3,17-Dion, 4-O-Valeryl-Androsten-3,17-Dion, 4-O-Caproyl-Androsten-3,17-Dion oder 4-O-Enantyl-Androsten-3,17-Dion ist.

7. Verwendung nach einem der vorhergehenden Ansprüche, **dadurch gekennzeichnet, dass** der Steroid-Carbonsäureester in einer Zusammensetzung in einer Menge von 0,001 bis 10 Gew.% bezogen auf die Gesamtmenge der Zusammensetzung enthalten ist.

8. Verwendung nach einem der vorhergehenden Ansprüche, **dadurch gekennzeichnet, dass** die Substanz in einer Zusammensetzung enthalten ist, die als Salbe, Creme, Gel, Öl oder Emulsion bzw. Lotion formuliert ist.

9. Verwendung eines Steroid-4-Carbonsäureesters der Androst-4-en-dione, mit einer Acylgruppe des Carbonsäureesters 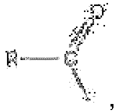
wobei R ausgewählt ist aus Alkyl mit mindestens zwei Kohlenstoffatomen oder Cycloalkyl,
oder Verwendung einer diese Steroid-Carbonsäureester enthaltenden Zusammensetzung bei lokal topischer Applikation für kosmetische Zwecke zur Reduktion von Cellulite, allgemeinen Hautstraffung, Abmilderung von Dehnungsstreifen, Reduktion der Größe von Narben sowohl vor und nach deren Entstehung, **dadurch gekennzeichnet, dass** ein Steroid-Carbonsäureester bzw. eine Zusammensetzung verwendet wird, wie in einem der Ansprüche 1 bis 8 definiert.

10. Zusammensetzung zur Verwendung bei lokal topischer Applikation für kosmetische Zwecke, **dadurch gekennzeichnet, dass** sie mindestens einen Steroid-Carbonsäureester, wie in einem der Ansprüche 1 bis 6 definiert, in einer Formulierung als Salbe, Creme, Gel, Öl, Emulsion oder Lotion enthält.

11. Zusammensetzung nach Anspruch 10, **dadurch gekennzeichnet, dass** der Steroid-Carbonsäureester in der Zusammensetzung in einer Menge von 0,001 bis 10 Gew.% bezogen auf die Gesamtmenge der Zusammensetzung enthalten ist.

12. Zusammensetzung nach Anspruch 10 , **dadurch gekennzeichnet, dass** der Steroid-Carbonsäureester in der Zusammensetzung in einer Menge von 0,001 bis 5 Gew.% bezogen auf die Gesamtmenge der Zusammensetzung enthalten ist.

13. Steroid-4-Carbonsäureester der Androst-4-en-dione, mit einer Acylgruppe des Carbonsäureesters 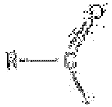
wobei R ausgewählt ist aus Alkyl mit mindestens zwei Kohlenstoffatomen oder Cycloalkyl,
oder eine diese Steroid-Carbonsäureester enthaltende Zusammensetzung zur Verwendung bei lokal topischer Applikation für dermatologische Zwecke, **dadurch gekennzeichnet, dass** der Steroid-Carbonsäureester ein Derivat des 4-Hydroxyandrostendions gemäß einem der Ansprüche 1 bis 6 ist.

14. Steroid-4-Carbonsäureester der Androst-4-en-dione zur Verwendung nach Anspruch 13 **dadurch gekennzeichnet, dass** der Steroid-Carbonsäureester 4-O-Propionyl-Androst-4-en-3,17-Dion mit der Formel 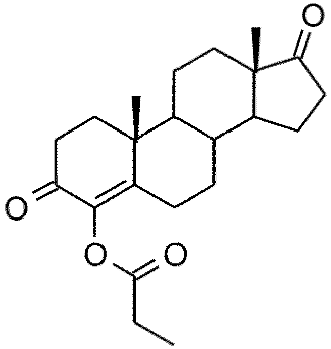 ist.

15. Zusammensetzung zur Verwendung nach Anspruch 13 **dadurch gekennzeichnet, dass** der Steroid-Carbonsäureester in der Zusammensetzung in einer Menge von 0,001 bis 10 Gew.% bezogen auf die Gesamtmenge der Zusammensetzung enthalten ist.

16. Zusammensetzung zur Verwendung nach Anspruch 13 oder 15 **dadurch gekennzeichnet, dass** der Steroid-Carbonsäureester in einer Zusammensetzung enthalten ist, die als Salbe, Creme, Gel, Öl oder Emulsion bzw. Lotion formuliert ist.

17. Steroid-4-Carbonsäureester der Androst-4-en-dione, mit einer Acylgruppe des Carbonsäureesters 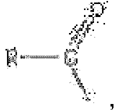
wobei R ausgewählt ist aus Alkyl mit mindestens zwei Kohlenstoffatomen oder Cycloalkyl,
oder eine diese Steroid-Carbonsäureester enthaltende Zusammensetzung zur Verwendung bei lokal topischer Applikation für dermatologische Zwecke zur Abmilderung von Hautirritationen auf allergischer Basis oder aufgrund von Insektenstichen oder UV- oder Roentgeneinstrahlung und allgemeinem Juckreiz der Haut, **dadurch gekennzeichnet, dass** ein Steroid-Carbonsäureester bzw. eine Zusammensetzung verwendet wird, wie in einem der Ansprüche 1 bis 8 definiert.

## Claims

1. Use of a steroid-4-carboxylic acid ester of the androst-4-ene-diones,
with one acyl group of the carboxylic acid ester 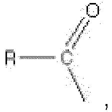
where R is selected from alkyl with at least two carbon atoms or cycloalkyl,
or with the use of a composition containing these steroid carboxylic acid esters for local topical application for cosmetic purposes, **characterised in that** the steroid carboxylic acid ester is a derivative of the 4-hydroxyandrostenedione.

2. Use in accordance with Claim 1, **characterised in that** the acyl group of the carboxylic acid ester is free of polar substitutes.

3. Use in accordance with one of the preceding claims, **characterised in that** the radical R of the acyl group is an alkyl radical with at least two carbon atoms and that the alkyl radical is either unbranched or branched.

4. Use in accordance with one of the preceding claims, **characterised in that** the radical R of the acyl group is an alkyl radical with at least two carbon atoms and that the alkyl radical has been selected from ethyl, propyl, isopropyl, butyl, sec-butyl (1-methylpropyl), isobutyl (2-methylpropyl), tert-butyl (1,1-dimethylethyl), pentyl, hexyl and structural isomers of pentyl or hexyl.

5. Use in accordance with one of the preceding claims, **characterised in that** the radical R of the acyl group is a cycloalkyl radical and that the cycloalkyl radical has been selected from cyclopropane, cyclopentane or cyclohexane.

6. Use in accordance with one of the preceding claims, **characterised in that** the steroid carboxylic acid ester is 4-O-propionyl-androstene-3,17-dione, 4-O-butyryl-androstene-3,17-dione, 4-O-valeryl-androstene-3,17-dione, 4-O-caproyl-androstene-3,17-dione or 4-O-enantyl-androstene-3,17-dione.

7. Use in accordance with one of the preceding claims, **characterised in that** the steroid carboxylic acid ester forms part of a composition in a ratio of 0.001 to 10% by weight of the total volume of the composition.

8. Use in accordance with one of the preceding claims, **characterised in that** the substance forms part of a composition formulated as an ointment, cream, gel, oil, emulsion or lotion.

9. Use of a steroid-4-carboxylic acid ester of the androst-4-ene-diones,
with one acyl group of the carboxylic acid ester 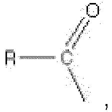
where R is selected from alkyl with at least two carbon atoms or cycloalkyl,
or the use of a composition containing these steroid carboxylic acid esters, for local topical application for cosmetic purposes for the reduction of cellulite, general skin firming, the reduction of stretch marks, a reduction in the size of scars both before and after their formation, **characterised in that** a steroid carboxylic acid ester or a composition is used, as defined in one of Claims 1 to 8.

10. A composition for use as a local topical application for cosmetic purposes, **characterised in that** it contains at least one steroid carboxylic acid ester, as defined in one of Claims 1 to 6, in a formulation as an ointment, cream, gel, oil, emulsion or lotion.

11. A composition in accordance with Claim 10, **characterised in that** the steroid carboxylic acid ester forms part of a composition in a ratio of 0.001 to 10% by weight of the total volume of the composition.

12. A composition in accordance with Claim 10, **characterised in that** the steroid carboxylic acid ester forms part of a composition in a ratio of 0.001 to 5% by weight of the total volume of the composition.

13. Steroid-4-carboxylic acid ester of the androst-4-ene-diones,
with one acyl group of the carboxylic acid ester 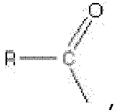
where R is selected from alkyl with at least two carbon atoms or cycloalkyl,
or a composition containing these steroid carboxylic acid esters for use in local topical application for dermatological purposes, **characterised in that** the steroid carboxylic acid ester is a derivative of the 4-hydroxyandrostenedione according to one of the Claims 1 to 6.

14. Steroid-4-carboxylic acid ester of the androst-4-ene-diones for use in accordance with Claim 13, **characterised in that** the steroid carboxylic acid ester is 4-O-propionyl-androst-4-ene-3,17-dione with the formula

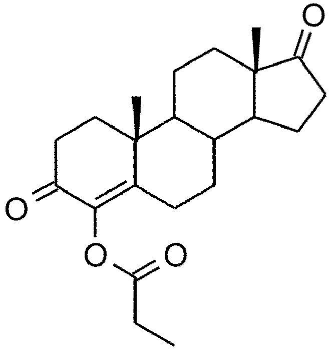

15. A composition for use in accordance with Claim 13, **characterised in that** the steroid carboxylic acid ester forms part of a composition in a ratio of 0.001 to 10% by weight of the total volume of the composition.

16. A composition for use in accordance with Claim 13 or 15, **characterised in that** the steroid carboxylic acid ester forms part of a composition formulated as an ointment, cream, gel, oil, emulsion or lotion.

17. Steroid-4-carboxylic acid ester of the androst-4-ene-diones,
with one acyl group of the carboxylic acid ester 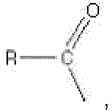
where R is selected from alkyl with at least two carbon atoms or cycloalkyl,
or a composition containing these steroid carboxylic acid esters for use in local topical application for dermatological purposes for a lessening of allergy-related skin irritations or irritations caused by insect bites, ultraviolet radiation or X-rays and general itchiness of the skin, **characterised in that** a steroid carboxylic acid ester or a composition is used, as defined in one of Claims 1 to 8.

## Revendications

1. Utilisation d'un ester d'acide stéroïde-carboxylique 4 de la 4-androstènedione,
avec un groupe acyle de l'ester d'acide carboxylique 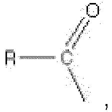
R étant sélectionné à partir d'alkyle avec au moins deux atomes de carbone ou de cycloalkyle,
ou utilisation d'une composition contenant ces esters d'acide stéroïde-carboxylique dans une application localement topique à des fins cosmétiques, **caractérisée en ce que** l'ester d'acide stéroïde-carboxylique est un dérivé de la 4-hydroxyandrostènedione.

2. Utilisation selon la revendication 1, **caractérisée en ce que** le groupe acyle de l'ester d'acide carboxylique est exempt de substituants polaires.

3. Utilisation selon l'une des revendications précédentes, **caractérisée en ce que** le reste R du groupe acyle est un reste alkyle avec au moins deux atomes de carbone et que le reste alkyle est ramifié ou non.

4. Utilisation selon l'une des revendications précédentes, **caractérisée en ce que** le reste R du groupe acyle est un reste alkyle avec au moins deux atomes de carbone et que le reste alkyle est sélectionné à partir d'éthyle, de propyle, d'isopropyle, de butyle, de sec-butyle (1-méthylpropyle), d'isobutyle (2-méthylpropyle), de tert-butyle (1,1-diméthyléthyle), de pentyle, d'hexyle ainsi que d'isomères de structure du pentyle ou de l'hexyle.

5. Utilisation selon l'une des revendications précédentes, **caractérisée en ce que** le reste R du groupe acyle est un reste cycloalkyle et que le reste cycloalkyle est sélectionné à partir de cyclopropane, cyclopentane ou cyclohexane.

6. Utilisation selon l'une des revendications précédentes, **caractérisée en ce que** l'ester d'acide stéroïde-carboxylique est une substance 4-O-propionyle-androstène-3,17-dione, 4-O-butyryle-androstène-3,17-dione, 4-O-valéryle-androstène-3,17-dione, 4-O-caproyle-androstène-3,17-dione ou 4-O-énantyle-androstène-3,17-dione.

7. Utilisation selon l'une des revendications précédentes, **caractérisée en ce que** l'ester d'acide stéroïde-carboxylique est contenu dans une composition à raison de 0,001 à 10 % en poids rapporté à la quantité totale de la composition.

8. Utilisation selon l'une des revendications précédentes, **caractérisée en ce que** la substance est contenue dans une composition formulée comme pommade, crème, gel, huile, émulsion ou lotion.

9. Utilisation d'un ester d'acide stéroïde-carboxylique 4 de la 4-androstènedione,
avec un groupe acyle de l'ester d'acide carboxylique 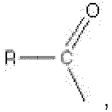
R étant sélectionné à partir d'alkyle avec au moins deux atomes de carbone ou de cycloalkyle
ou utilisation d'une composition contenant ces esters d'acide stéroïde-carboxylique dans une application localement topique à des fins cosmétiques pour réduire la cellulite, assurer un raffermissement général de la peau, estomper les vergetures, diminuer la taille de cicatrices avant et après leur formation, **caractérisé en ce qu'**un ester d'acide stéroïde-carboxylique ou une composition est utilisée, comme défini dans l'une des revendications 1 à 8.

10. Composition pour l'utilisation dans une application localement topique à des fins cosmétiques, **caractérisée en ce qu'**elle contient au moins un ester d'acide stéroïde-carboxylique, comme défini dans l'une des revendications 1 à 6, dans une formulation comme pommade, crème, gel, huile, émulsion ou lotion.

11. Composition selon la revendication 10, **caractérisée en ce que** l'ester d'acide stéroïde-carboxylique est contenu dans la composition à raison de 0,001 à 10 % en poids rapporté à la quantité totale de la composition.

12. Composition selon la revendication 10, **caractérisée en ce que** l'ester d'acide stéroïde-carboxylique est contenu dans la composition à raison de 0,001 à 5 % en poids rapporté à la quantité totale de la composition.

13. Ester d'acide stéroïde-carboxylique 4 de la 4-androstènedione,
avec un groupe acyle de l'ester d'acide carboxylique 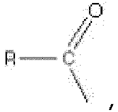
R étant sélectionné à partir d'alkyle avec au moins deux atomes de carbone ou de cycloalkyle,
ou une composition contenant ces esters d'acide stéroïde-carboxylique pour une utilisation dans une application localement topique à des fins dermatologiques, **caractérisée en ce que** l'ester d'acide stéroïde-carboxylique est un dérivé de la 4-hydroxyandrostènedione selon l'une des revendications 1 à 6.

14. Ester d'acide stéroïde-carboxylique 4 de la 4-androstènedione pour une utilisation selon la revendication 13, **caractérisé en ce que** l'ester d'acide stéroïde-carboxylique est une substance 4-O-propionyle-androst-4-ène-3,17-dione avec la formule

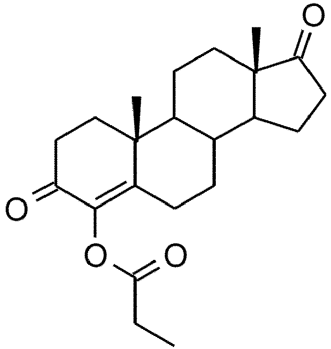

15. Composition pour une utilisation selon la revendication 13, **caractérisée en ce que** l'ester d'acide stéroïde-carboxylique est contenu dans la composition à raison de 0,001 à 10 % en poids rapporté à la quantité totale de la composition.

16. Composition pour une utilisation selon la revendication 13 ou 15, **caractérisée en ce que** l'ester d'acide stéroïde-carboxylique est contenu dans une composition formulée comme pommade, crème, gel, huile, émulsion ou lotion.

17. Ester d'acide stéroïde-carboxylique 4 de la 4-androstènedione,
avec un groupe acyle de l'ester d'acide carboxylique 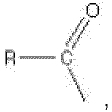
R étant sélectionné à partir d'alkyle avec au moins deux atomes de carbone ou de cycloalkyle
ou utilisation d'une composition contenant ces esters d'acide stéroïde-carboxylique dans une application localement topique à des fins dermatologiques pour réduire les irritations cutanées provenant d'une allergie ou de piqûres d'insecte, d'un rayonnement UV ou X et diminuer les démangeaisons cutanées générales, **caractérisé en ce qu'**un ester d'acide stéroïde-carboxylique ou une composition est utilisé(e), comme défini dans l'une des revendications 1 à 8.
